# EUROPEAN PATENT SPECIFICATION

(11) **EP 1 715 926 B1**
(45) Date of publication and mention of the grant of the patent: **29.11.2017**
(21) Application number: 05702990.2
(22) Date of filing: 16.02.2005
(51) Int. Cl.: A63B 69/00

(54) **Audio interval training device**
Audio-intervalltrainingsvorrichtung
Dispositif de pilotage d'intervalle audio

(30) Priority: 19.02.2004 US 546076 P
(43) Date of publication of application: 02.11.2006
(73) Proprietor: Koninklijke Philips N.V., 5656 AE Eindhoven (NL)
(72) Inventor: OGG, Felix Henric Govert, NL-5621 BA Eindhoven (NL); SIMON, David Peter Louis, NL-5621 BA Eindhoven (NL)
(74) Representative: Damen, Daniel Martijn
(86) International application number: PCT/IB2005/050586
(87) International publication number: WO 2005/082471

(56) References cited:
- EP-A- 1 101 511
- EP-A- 1 251 441
- EP-A- 1 512 370
- EP-A1- 1 512 370
- WO-A-2004/072767
- US-A1- 2002 091 049
- PICARD R W ET AL: "Affective wearables" WEARABLE COMPUTERS, 1997. DIGEST OF PAPERS., FIRST INTERNATIONAL SYMPOSIUM ON CAMBRIDGE, MA, USA 13-14 OCT. 1997, LOS ALAMITOS, CA, USA,IEEE COMPUT. SOC, US, 13 October 1997 (1997-10-13), pages 90-97, XP010251547 ISBN: 0-8186-8192-6

## Description

The present invention relates to a device and methods using an audio (music) signal, a parameter of a user, such as pulse rate (heartbeat), and musical listening devices for interval training. More particularly, the present invention relates to a device and method capable of selecting an audio signal corresponding to an appropriate tempo, for example, beats per minute (BPM) to achieve two interval exercise levels.

Numerous physical exercise programs and, in particular, running or jogging performed by individuals are significantly more effective in a particular range of heart beat rates. The required heart beat rates vary for the individuals with different ages and health factors. Moreover, individual heart beat rates that drop to a specific low level, result in physical exercises that can have only a slight influence on them. Further, individual heart beat rates that go beyond an upper limit level, result in exercises that can have a harmful influence on them. Accordingly, people who start physical exercise programs should exercise in amounts suitable for them according to their physical status.

Heart beat (or pulse rate) analysis devices are used as an exercise amount measurement. They measure heart beat rate (or pulse) of an individual in exercise in heart beats per minute (pulse), and compare the measured number with a standard table, so that one measures the physical exercise as weak, medium, or strong for oneself. Further, sports literature is available with specific tables for goals such as fat-burning/weight loss, energizing exercise, cardiovascular training or speed training. Depending on one's weight, height, body fat percentage, age, gender (etc.) one can find the ideal pulse pace for the goal at hand.

Particularly athletes, who want to track their improvements, and thus stay motivated to follow through, find heartbeat monitoring tools motivating and stimulating.

Conventional heart beat analysis devices, such as the Polar™ sportswatch, are known to give audible feedback (e.g. a beep) when the pulse of a user/athlete exceeds a target pulse threshold (upper or lower). It thereby signals the user to adjust the exercise strength accordingly to return to the appropriate training zone or level.

However, such conventional heartbeat analysis devices suffer from a number of limitations. For example, many users find the beeping annoying and as a result are known to turn the beeping off altogether. Further, other athletes use portable music playback devices, such as MP3 players, during exercise routines and cannot hear the beeping.

Further, listening to music while performing physical exercise is very popular. However, a problem of listening to music while exercising is that the music often has a tempo which is out of synchronization with the pace of the person exercising. Modem day pop music is generally supported by a clear drumbeat that can be followed in the pace of a physical exercise. Soldiers march to the beat of a song and in the same way Roman slaves rowed their boats to a drumbeat. Athletes enjoy moving along the beat of music playing, since this helps them in keeping a constant pace. Music is a powerful tool to increase the performance of athletes if they are sensitive to it.

Examples of training systems that may adapt an audio output to a users heart rate are disclosed in patent publications US2002091049; EP1251441; EP1101511 and the article "Affective Wearables" by R.W.Picard and J. Healey; 1997; Digest of Papers., First International Symposium on Cambridge, MA, USA 13-14 OCT. 1997, Los Alamitos, CA, USA,IEEE Comput. SOC, US, 13 October 1997 (1997-10-13), pages 90-97, XP010251547 ISBN: 0-8186-8192-6

Moreover, many users/athletes often use a technique known as interval training. During interval training the athlete alternates between two levels of exercise intensity (pace). For instance, the athlete sets a maximum heart rate and a minimum heart rate. The physical exercise or training consists of a small warming up phase, followed by several interval sets. First, the athlete puts in a maximum (e.g. 100%) effort, for example sprinting, until his heart rate reaches the target maximum. Thereafter, the athlete tries to recuperate as fast as possible, which means he exerts a mild exercise activity until his heart rate falls below the minimum target. These alternating steps are repeated several times.

In a similar way interval training can be performed based on time intervals rather than heart rate measurements. In that case the exercise is to put in a full effort (e.g. sprinting) for a small fixed period (e.g. 20 seconds) alternated by a second period of recuperation (e.g. 2 minutes). In this way a similar cycle is attained, be it less dependent on the actual state of the body.

Accordingly, there is a need for an apparatus that enables music supported effective interval training in a non-intrusive, motivating way.

The invention is defined by the independent claims.

The present invention is directed to a system and method for an audio interval training device and which enable a user to select two (a high and low) intensity levels for interval training and provides an audio signal that is correlated to the selected interval exercise program or routine to achieve the target performance levels. The two intensity levels can be based on two heartbeat rates.

The audio signals are ordered based on their tempo, for example, a measurement based on a beat per minute (BPM) value. The audio signal ordering can be conducted either by the heart beat interval training apparatus, or by an external device, such as a PC, and then transferred to the heart beat interval training apparatus. An audio signal is selected having a tempo (e.g. BPM) that paces the user to the current phase of exercise (e.g. sprinting). The audio signal comprises an MP3, WAV or WMA file, and the like, generally containing music.

In accordance with the principles of the present invention an audio interval training device is provided including a sensing unit to obtain a parameter of a user in physical exercise, the sensing unit being a heart rate monitor and the parameter being the user's heart rate as determined by the processing unit using a received heart rate from a heart rate monitor, a memory to store a plurality of audio signals having predetermined beat per minute values; and a processing unit configured to (1) receive a first and second target parameter value being a high heart rate target value and a low heart rate target value respectively ,(2) select a first and second audio signal having a high tempo and a low tempo, respectively, (3) alternatively rendering the first and second audio signals, wherein a respective audio signal is rendered to the user corresponding to the first and second target parameter value, as determined by the processing unit using the parameter from the sensing unit; wherein the processing unit is arranged to repeatedly render the first audio signal to the user until the user's heart rate reaches or exceeds the first target heart rate and then to render the second audio signal until the user's heart rate reaches or falls below the second target heart rate and then to render the first audio signal.

The present invention is more fully understood by reference to the following detailed description of a preferred embodiment in combination with the drawings identified below.
FIG. 1 is a view for showing a audio interval training device according to an embodiment of the present invention;
FIG. 2 is a block diagram for showing an internal structure of the processing unit FIG. 1;
FIG. 3 is a flow chart for showing a process of the heart rate audio interval training device of FIG. 1; and
FIG. 4 is a flow chart illustrating the interval training operation steps of heart rate audio interval training.

In the following description, for purposes of explanation rather than limitation, specific details are set forth such as the particular architecture, interfaces, techniques, etc., in order to provide a thorough understanding of the present invention. However, it will be apparent to those skilled in the art that the present invention may be practiced in other embodiments that depart from these specific details. As used herein the terms "pulse" means a heart-beat rate, "tempo" of an audio signal is, for example in BPM, and "pace" refers to an exercise tempo.

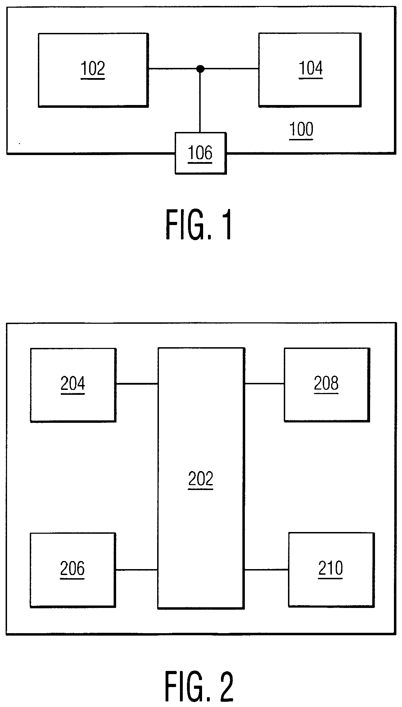

Referring to the drawings and, in particular, Fig. 1, there is shown an audio interval training device in accordance with the present invention generally represented by reference numeral 100.

As shown in FIG. 1, the present invention includes a processing unit 102, a sensing unit 104, for example a heart rate monitor or timer device, (hereinafter for exemplary purposes referred to as a "heart rate monitor 104"), and input/output port 106. The processing unit 102 performs the functions of: (1) receiving and storing parameter information of a user in physical exercise (hereinafter for exemplary purposes referred to as "heart rate"), (2) storing BPM rated audio signals and (3) selecting an audio signal with a particular BPM according to user's exercise program. The parameter information of a user in physical exercise may include any parameter related to a user physical status or condition, for example, time-intervals, heart rate or a step-rate measurement, speed measurement (in m/s), for example using the electronic speed sensor on sports bicycles, rowing trainers or any other kind of digital fitness equipment, the sit-up speed from a sit-up trainer equipped with a sensor to count the number of sit-ups per minute, similarly a sensor on a strap-on belt on an ankle/wrist/head/waist etc. (for aerobics ground exercises), providing a rating. In addition, processing unit 102 may also digitize received broadcast signals and encode them according to a particular coding scheme (e.g. MP3, WAV, MPEG-4 and the like) and BPM-rate received audio signals. Alternatively, as well be apparent to those skilled in the art, the processing unit 102 may also include an integrated sensing unit.

The processing unit measures a user's exercise effects by receiving and analyzing heart rate information of a user in physical exercise. For example, data analysis is performed for a change rate of the number of heart beats by time (second/minute/hour), a change rate of the number of heart beats by unit time (day/month/year), a suitable exercise intensity proposal based on a change of the number of heart beats, a proposal of the objective heart beats (a proper exercise amount) based on ages, gender, heights, and weights, an indication of an exercise improvement degree based on the change of the number of heart beats, a total amount of calories consumed during exercise, what percent of the total consumed calories amount comes from the body fat, and so on.

The processing unit 102 may be manufactured in a variety of designs, such as: (1) to be worn on the wrist like a wrist watch in one body with the heart rate monitor 104, (2) to be attached on the front of the user's sporting coat, (3) to be hung around the neck of a user by a string like a stop watch, and (4) to be integrated in a wearable fabric or clothing-type device. In particular, the use of integrated electronic and conductive fibers in various sewn or woven fabrics used as conductive traces, bio-sensors, electrodes, and other wearable electronic devices are well known. For example, in the case of a Wearable Heart Rate Monitor (WHRM) device for general sport applications, the electrodes can be fully made of fabric and can be fully integrated into a garment such as a running top. The electronics though that collect the data from the electrodes and transmit them wirelessly to a watch or similar device are contained in a separate small unit which can be attached onto the garment in such a way that it can make good electrical contact with the fabric electrodes.

The heart rate monitor 104 is connected to the processing unit 102 in a wire, wireless or wearable fabric way, and performs a function of measuring heart beats of a user in physical exercise and transferring the sampled heart beats to the processing unit 102. The heart rate monitor can be any conventional unit, for example the Polar ™ Sportswatch, by Polar Electronics. Such heart rate monitor 104 is carried on a wrist like a wrist watch.

The input/output port 106 is connected to the processing unit 102 and heart rate monitor for exchanging data, and includes the functions of transferring digitalized music files to the processor 102 and outputting audio signals, selected based on their tempo (e.g. their BPM rating).

The processing unit enables heart rate interval training. In particular, two separate audio signals (e.g. MP3 songs) are selected by a user via input/output port 106. The first audio signal provides a first pacing level for an upper (or maximum) heart rate. The second audio signal provides a second pacing level for a lower (or minimum) heart rate. During a fist phase of the interval training the first audio signal is rendered to a user to increase the user's heart rate. During a second phase of the interval training the second audio signal is rendered to the user to decease the user's heart rate. Alternating between the two audio signals is performed instantaneously (optionally with a few seconds of cross-fade). The rendered audio signal is paused, and the other audio signal is continued where it was paused the previous time. The heart rate monitor 104 is used to determine when to alternate the audio signals. Advantageously, the user obtains a clear indication, through the differing tempo audio signals, to exercise at either maximum or minimal intensity.

FIG. 2 is a component diagram of the processing unit 102 of FIG. 1 according to an embodiment of the present invention.

As shown in FIG. 2, the processing unit according to the present invention has a control unit 202, an output unit 204, a storage unit 206, a sensing signal receiving unit 208, and an input unit 210. The control unit 202 includes a conventional microcomputer and a digital signal processor (DSP) (not shown). The output unit 204 includes an audio output unit and may include an indication unit (not shown). The sensing signal receiving unit 208 receives sensing information (below by example referred to as heart rate) from the sensing unit 104 and transfers it to the control unit 202. As indicated above, a conventional heart rate monitor can also be integrated into the processing unit.

Storage unit 206 contains a group of BPM categorized audio signals, for example MP3s. In addition, storage unit 206 may contain programmed exercise routines or target exercise levels, as further described below, which are received using input unit 210.

Tempo-relative ordering of the audio signals can be performed by the processing unit or off-line and downloaded to the heart rate interval device. The BPM categorized audio signals can be input into the storage unit 206 using any conventional manner (e.g. downloaded from a PC, wirelessly transmitted, etc.) A conventional tool that does automatic (off-line) BPM analysis upon audio files to measure the musical tempo, (as well as dynamic (on-line) tempo adjustments, discussed below) is the PCDJ-Red product from Visiosonic, (see e.g. http://www.pcdj.com/products/Red.asp and http://www.curiousdjs.com/pcdj.html). This tool will determine the average BPM of a song to an accuracy of 0.01 BPM (such as 86.56 beats per minute). Once an audio signal is tempo-analyzed, the BPM value it is stored with the audio signal, for example in the header of the audio signal. In particular, in the case of MP3 files, the BPM value is stored in the MP3 file, as an ID3v2 BPM tag, which can be read by other applications subsequently. Thus, for example, MP3s can be downloaded to the device that are searched (e.g. on the Internet) for their ID3v2 BPM tag values. If no value for their BPM is available, it is generated by using BPM analysis algorithms in the device.

The control unit 202 stores the heart rate received into a storage unit 206, analyzes the number of heart beats received, compares it with a predetermined reference value (e.g. target level or programmed exercise program), and decides whether to either (1) toggle intensity phase (to high/low intensity) by selecting and consequently rendering a newly selected (or previously paused) audio signal having a tempo corresponding to the target intensity phase, or (2) to remain in the current intensity phase by continuing the rendering of the current audio signal or by selecting a similar tempo (BPM) audio signal. In either case rendering of the audio signal is performed using output unit 204.

The appropriate heart rates the user should attain are predetermined by the user or by any other conventional manner. Accordingly, an objective number of heart beats as a reference value range which can evaluate what extent of heart beats corresponds to a high-strength exercise, a medium-strength exercise, or a low-strength exercise. Moreover, established values for heart rates during physical exercise can be used that factor the age, sex, height, weight, etc., of a user. In addition, the processing unit can store (in storage unit 206) programmed training routines, using the above factors, for a user wherein heart rate levels are established. The programmed training routines can be input into the processing unit in any conventional manner. For example, a heartbeat sensor, such as the Polar™ sportswatch, can be manually programmed with a training zone (two heart beat values).

For example, the control unit 202 receives a heart rate of a user (an individual in physical exercise) from the sensing receiving unit 208, compares the received heart rate with the predetermined objective heart rate, and determines an appropriate tempo/BPM audio signal to output to help achieve the desired user heart rate. When a particular audio signal, or song/music ends, while the user is in an exercise program, another audio signal with a similar tempo/BPM is selected from the store group of audio signals.

The processing device is also enabled to receive input from a user, using the input unit 210, for example a button, during exercise to denote particular audio signals or songs that support the exercise or training. Also, the user can use the input unit 210 to denote favorite audio signals. This in turn will enhance the probability that the particular audio signal with be selected during a later exercise session. For example, once an audio signal is denoted, if an audio signal is needed in the future within a predetermined range (e.g. +/- 10%) of the denoted audio signal's BPM, it will be selected.

In a similar manner, the user can store an indication of the type of exercise, for example, the type of sport, running, rowing, cycling, etc., along with the denoted audio signal. Thus, the processing device records the most appropriate audio signal for each type of sport or exercise for a user. This, in turn, shortens the time to select an appropriate audio signal.

The sensing receiving unit 208 receives sensing data from the sensing unit 104 in a wired or wireless way. In case of wired, data is received through a certain cable, and, in case of wireless, receptions are carried out by using a wireless signal such as an RF signal and the like.

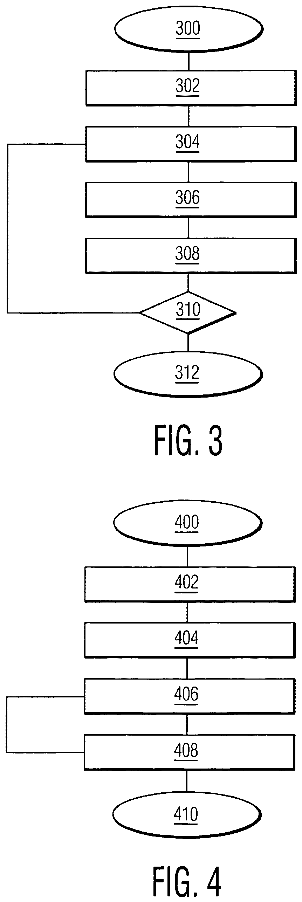

FIG. 3 is a flow chart illustrating the operation steps of reference-based audio interval pacing.

As shown in FIG. 3, the process begins (start 300) with a user selecting an exercise level(s) or program in step 302. In step 304, the processing unit 102 receives heart rate information from the heart rate monitor and stores it in storage unit 206. The control unit 202 analyzes the receive heart rate information and compares it to the selected exercise levels in step 306. In particular, the control unit determines the heart rate of the user and at what stage or level the user is in the selected exercise program and determines whether the user needs to increase, decrease or maintain the current heart rate. In step 308, the control unit 202 selects an audio signal form storage unit 206 in accordance with the determination of step 306 and provides it to the user. In step 310, process determines whether the selected exercise program has ended (end 312)or returns to the step 304.

FIG. 4 is a flow chart illustrating the interval training operation steps the audio interval training.
The interval training operation is initiated (START 400) by a user using the input/output port 106. The user sets a low and a high target parameter value (i.e. high and low heart rate) in step 402. In step 404, the processing unit 102 selects the first (high tempo) and second (low tempo) audio signals based on their BPM values. For example, all audio signals are ordered based on their BPM, and the processing unit selects two songs: one out of the set of 10% slowest and one out of the fastest 10%. In addition, the processing unit can use the classification of songs (audio signals) by their tempo: slow, normal, up-tempo, and fast. Thus, selecting a song from the slow, and one from the up-tempo or even fast class. In step 406, the first (fast) audio signal is then rendered to the user until the user's heart rate reaches or exceeds the high target value, as determined by the processing unit 102 using a received heart rate from the heart rate monitor 104. Then the first audio signal is paused and the second (slow) audio signal is rendered, possibly with a few seconds of crossfading. In step 408, when the user's heart rate reaches or falls below the low BPM target value, as determined by the processing unit 102 using a received heart rate from the heart rate monitor 104, the second audio signal is paused and the first audio signal is continued. This process is repeated until the user indicates an end (END 410). When an audio signal, such as a song, ends, another audio signal of similar tempo (BPM) can be rendered, or the audio signal can be repeated.

The preceding and following merely illustrates the principles of the invention. It will thus be appreciated that those skilled in the art will be able to devise various arrangements which, although not explicitly described or shown herein, embody the principles of the invention and are included within its scope. Additionally, it is intended that such equivalents include both currently known equivalents as well as equivalents developed in the future, i.e., any elements developed that perform the same function, regardless of structure.

The functions of the various elements shown in FIGs. 1 and 2, may be provided through the use of dedicated hardware as well as hardware capable of executing software in association with appropriate software. When provided by a processor, the functions may be provided by a single dedicated processor, by a single shared processor, or by a plurality of individual processors, some of which may be shared. Moreover, explicit use of the term "processor", "server" or "controller" should not be construed to refer exclusively to hardware capable of executing software, and may implicitly include, without limitation, digital signal processor (DSP) hardware, read-only memory (ROM) for storing software, random access memory (RAM) and non-volatile storage. Other hardware, conventional and/or custom, may also be included.

In the claims hereof any element expressed as a means for performing a specified function is intended to encompass any way of performing that function including, for example, a) a combination of circuit elements which performs that function or b) software in any form, including, downloadable or retrofit software or the like, combined with appropriate circuitry for executing that software to perform the function. The invention as defined by such claims resides in the fact that the functionalities provided by the various recited means are combined and brought together in the manner which the claims call for. Applicant thus regards any means which can provide those functionalities as equivalent as those shown herein.

## Claims

1. An audio interval training device (100), comprising:
a sensing unit (104) to obtain a parameter of a user in physical exercise, the sensing unit (104) being a heart rate monitor (104) and the parameter being the user's heart rate as determined by a processing unit (102) using a received heart rate from the heart rate monitor (104);
a memory (206) to store a plurality of audio signals, each having a predetermined tempo value; and the processing unit (102) configured to i) receive a first and second target parameter value being a high heart rate target value and a low heart rate target value respectively, ii) select a first and second audio signal having a high tempo and a low tempo, respectively,
the device being **characterized by** the processing unit being further configured to iii) alternatively render the first and second audio signals, wherein a respective audio signal is rendered to the user corresponding to the first and second target parameter value, as determined by the processing unit (102) using the parameter from the sensing unit (104); wherein the processing unit (102) is arranged to repeatedly render the first audio signal to the user until the user's heart rate reaches or exceeds the first target heart rate and then to render the second audio signal until the user's heart rate reaches or falls below the second target heart rate and then to render the first audio signal.

2. The audio interval training device (100) as claimed in claim 1, wherein the tempo is a beat per minute value.

3. The audio interval training device (100) as claimed in claim 1, wherein the sensing unit (104) and the processing unit (102) are connected in a wired or wireless way.

4. The audio interval training device (100) as claimed in claim 1, wherein the first and second target parameter value include target parameter value selected by a user or a programmed exercise routine.

5. The audio interval training device (100) as claimed in claim 1, wherein the audio signals are annotated with their beat per minute value.

6. The audio interval training device (100) as claimed in claim 1, wherein the tempo values of the plurality of audio signal are determined either by the audio interval training device (100), or by an external device and transferred to the audio interval training device (100).

7. The audio interval training device (100) as claimed in claim 1, wherein the audio signals are encoded in an MP3, WAV, MPEG-4, WMA or AAC format.

8. A method of operation for an audio interval training device (100), the method comprising steps of:
receiving a first and second target parameter value being a high heart rate target value and a low heart rate target value respectively;
receiving a parameter of a user in physical exercise from a sensing unit (104), the sensing unit (104) being a heart rate monitor (104) and the parameter being the user's heart rate as determined by a the processing unit (102) using a received heart rate from the heart rate monitor (104);
selecting a first and second audio signal having a high tempo and a low tempo, respectively; and
the method being **characterized by** further comprising:
alternatively rendering the first and second audio signal to a user, wherein a respective audio signal is rendered to the user corresponding to the first and second target parameter value, as determined by the processing unit (102) using the parameter from the sensing unit (104); wherein the rendering comprises repeatedly rendering the first audio signal to the user until the user's heart rate reaches or exceeds the first target heart rate and then rendering the second audio signal until the user's heart rate reaches or falls below the second target heart rate and then rendering the first audio signal.

9. The method as claimed in claim 8, further comprising the step of, a user, selecting the first and second target parameter value from a group of predetermined target parameter value or a programmed exercise routine that includes the first and second target parameter value.

10. The method as claimed in claim 8, wherein the audio signals are encoded in an MP3, WAV, MPEG-4 or WMA format.

11. The method as claimed in claim 8, further comprising the step of, selecting a third and/or forth audio signal having respective tempos similar to the first and second audio signals.

12. The method as claimed in claim 9, further comprising the step of, at a predetermined time, rendering the third and forth audio signals in place of the first and second audio signals respectively.

## Patentansprüche

1. Audio-Intervalltrainingsvorrichtung (100), umfassend:
eine Sensoreinheit (104), um einen Parameter eines Benutzers bei körperlicher Betätigung zu erhalten, wobei die Sensoreinheit (104) ein Herzfrequenzüberwachungsgerät (104) ist und der Parameter die Herzfrequenz des Benutzers, wie durch eine Verarbeitungseinheit (102) unter Verwendung einer von dem Herzüberwachungsgerät (104) empfangenen Herzfrequenz ermittelt, darstellt;
einen Speicher (206), um eine Vielzahl von Audiosignalen zu speichern, die jeweils einen vorgegebenen Tempowert aufweisen; sowie
die Verarbeitungseinheit (102), die so konfiguriert ist, dass sie i) einen ersten und zweiten Zielparameterwert empfängt, der einen hohen Herzfrequenzzielwert beziehungsweise einen niedrigen Herzfrequenzzielwert darstellt, ii) ein erstes und zweites Audiosignal mit einem hohen Tempo beziehungsweise einem geringen Tempo auswählt,
wobei die Vorrichtung **dadurch gekennzeichnet ist, dass** die Verarbeitungseinheit weiterhin so konfiguriert ist, dass sie iii) alternativ das erste und zweite Audiosignal wiedergibt, wobei ein jeweiliges Audiosignal dem Benutzer entsprechend dem ersten und zweiten Zielparameterwert, wie durch die Verarbeitungseinheit (102) unter Verwendung des Parameters von der Sensoreinheit (104) ermittelt, wiedergegeben wird; wobei die Verarbeitungseinheit (102) so eingerichtet ist, dass sie dem Benutzer wiederholt das erste Audiosignal wiedergibt, bis die Herzfrequenz des Benutzers die erste Zielherzfrequenz erreicht oder überschreitet, und sie danach das zweite Audiosignal wiedergibt, bis die Herzfrequenz des Benutzers die zweite Zielherzfrequenz erreicht oder diese unterschreitet, and sie anschließend das erste Audiosignal wiedergibt.

2. Audio-Intervalltrainingsvorrichtung (100) nach Anspruch 1, wobei es sich bei dem Tempo um einen Schlag pro Minutenwert handelt.

3. Audio-Intervalltrainingsvorrichtung (100) nach Anspruch 1, wobei die Sensoreinheit (104) und die Verarbeitungseinheit (102) verdrahtet oder drahtlos miteinander verbunden sind.

4. Audio-Intervalltrainingsvorrichtung (100) nach Anspruch 1, wobei der erste und zweite Zielparameterwert einen Zielparameterwert enthalten, der von einem Benutzer oder einem programmierten Trainingsprogramm ausgewählt wird.

5. Audio-Intervalltrainingsvorrichtung (100) nach Anspruch 1, wobei die Audiosignale mit ihrem Schlag pro Minutenwert annotiert werden.

6. Audio-Intervalltrainingsvorrichtung (100) nach Anspruch 1, wobei die Tempowerte der Vielzahl von Audiosignalen entweder durch die Audio-Intervalltrainingsvorrichtung (100) oder durch eine externe Vorrichtung ermittelt und zu der Audio-Intervalltrainingsvorrichtung (100) übertragen werden.

7. Audio-Intervalltrainingsvorrichtung (100) nach Anspruch 1, wobei die Audiosignale in einem MP3-, WAV-, MPEG-4-, WMA- oder AAC-Format codiert sind.

8. Verfahren zum Betrieb einer Audio-Intervalltrainingsvorrichtung (100), wobei das Verfahren die folgenden Schritte umfasst, wonach:
ein erster und zweiter Zielparameterwert empfangen werden, die einen hohen Herzfrequenzzielwert beziehungsweise einen niedrigen Herzfrequenzzielwert darstellen;
von einer Sensoreinheit (104) ein Parameter eines Benutzers bei körperlicher Betätigung empfangen wird, wobei die Sensoreinheit (104) ein Herzfrequenzüberwachungsgerät (104) ist und der Parameter die Herzfrequenz des Benutzers, wie durch eine Verarbeitungseinheit (102) unter Verwendung einer von dem Herzüberwachungsgerät (104) empfangenen Herzfrequenz ermittelt, darstellt;
ein erstes und zweites Audiosignal ausgewählt werden, die ein hohes Tempo beziehungsweise ein geringes Tempo aufweisen; und
wobei das Verfahren **dadurch gekennzeichnet ist, dass** weiterhin:
alternativ einem Benutzer das erste und zweite Audiosignal wiedergegeben werden, wobei ein jeweiliges Audiosignal dem Benutzer entsprechend dem ersten und zweiten Zielparameterwert, wie durch die Verarbeitungseinheit (102) unter Verwendung des Parameters von der Sensoreinheit (104) ermittelt, wiedergegeben wird; wobei die Wiedergabe beinhaltet, dass dem Benutzer wiederholt das erste Audiosignal wiedergegeben wird, bis die Herzfrequenz des Benutzers die erste Zielherzfrequenz erreicht oder überschreitet, und danach das zweite Audiosignal wiedergegeben wird, bis die Herzfrequenz des Benutzers die zweite Zielherzfrequenz erreicht oder diese unterschreitet, and anschließend das erste Audiosignal wiedergegeben wird.

9. Verfahren nach Anspruch 8, das weiterhin den Schritt umfasst, wonach ein Benutzer den ersten und zweiten Zielparameterwert aus einer Gruppe von vorgegebenen Zielparameterwerten oder einem programmierten Trainingsprogramm, das den ersten und zweiten Zielparameterwert enthält, auswählt.

10. Verfahren nach Anspruch 8, wobei die Audiosignale in einem MP3-, WAV-, MPEG-4- oder WMA-Format codiert werden.

11. Verfahren nach Anspruch 8, das weiterhin den Schritt umfasst, wonach ein drittes und/oder viertes Audiosignal mit jeweiligen, dem ersten und zweiten Audiosignal ähnlichen Tempos ausgewählt werden/wird.

12. Verfahren nach Anspruch 9, das weiterhin den Schritt umfasst, wonach zu einem vorgegebenen Zeitpunkt das dritte und vierte Audiosignal anstelle des ersten beziehungsweise zweiten Audiosignals wiedergegeben werden.

## Revendications

1. Dispositif de pilotage d'intervalle audio (100), comprenant :
une unité de détection (104) pour obtenir un paramètre d'un utilisateur lors d'un exercice physique, l'unité de détection (104) étant un moniteur de fréquence cardiaque (104) et le paramètre étant la fréquence cardiaque de l'utilisateur telle que déterminée par une unité de traitement (102) utilisant une fréquence cardiaque reçue depuis le moniteur de fréquence cardiaque (104) ;
une mémoire (206) pour stocker une pluralité de signaux audio, chacun ayant une valeur de tempo prédéterminée ; et
l'unité de traitement (102) étant configurée pour i) recevoir une première et une deuxième valeur de paramètre cible qui sont respectivement une valeur cible de fréquence cardiaque élevée et une valeur cible de fréquence cardiaque basse, ii) sélectionner un premier et un deuxième signal audio ayant respectivement un tempo élevé et un tempo bas,
le dispositif étant **caractérisé en ce que** l'unité de traitement est en outre configurée pour iii) rendre alternativement les premier et deuxième signaux audio, dans lequel un signal audio respectif est rendu à l'utilisateur correspondant à la première et à la deuxième valeur de paramètre cible, telles que déterminées par l'unité de traitement (102) utilisant le paramètre provenant de l'unité de détection (104) ; dans lequel l'unité de traitement (102) est agencée pour rendre de façon répétée le premier signal audio à l'utilisateur jusqu'à ce que la fréquence cardiaque de l'utilisateur atteigne ou dépasse la première fréquence cardiaque cible puis rendre le deuxième signal audio jusqu'à ce que la fréquence cardiaque de l'utilisateur atteigne ou tombe sous la deuxième fréquence cardiaque cible puis rendre le premier signal audio.

2. Dispositif de pilotage d'intervalle audio (100) selon la revendication 1, dans lequel le tempo est une valeur de battements par minute.

3. Dispositif de pilotage d'intervalle audio (100) selon la revendication 1, dans lequel l'unité de détection (104) et l'unité de traitement (102) sont connectées d'une manière câblée ou sans fil.

4. Dispositif de pilotage d'intervalle audio (100) selon la revendication 1, dans lequel les première et deuxième valeurs de paramètre cibles incluent une valeur de paramètre cible sélectionnée par un utilisateur ou une routine d'exercices programmés.

5. Dispositif de pilotage d'intervalle audio (100) selon la revendication 1, dans lequel les signaux audio sont annotés de leur valeur de battements par minute.

6. Dispositif de pilotage d'intervalle audio (100) selon la revendication 1, dans lequel les valeurs de tempo de la pluralité de signaux audio sont déterminées soit par le dispositif de pilotage d'intervalle audio (100), soit par un dispositif externe et transférées au dispositif de pilotage d'intervalle audio (100).

7. Dispositif de pilotage d'intervalle audio (100) selon la revendication 1, dans lequel les signaux audio sont codés dans un format MP3, WAV, MPEG-4, WMA ou AAC.

8. Procédé de fonctionnement d'un dispositif de pilotage d'intervalle audio (100), le procédé comprenant les étapes consistant à :
recevoir des première et deuxième valeurs de paramètre cibles qui sont respectivement une valeur cible de fréquence cardiaque élevée et une valeur cible de fréquence cardiaque basse ;
recevoir un paramètre d'un utilisateur lors d'un exercice physique provenant d'une unité de détection (104), l'unité de détection (104) étant un moniteur de fréquence cardiaque (104) et le paramètre étant la fréquence cardiaque de l'utilisateur telle que déterminée par une unité de traitement (102) utilisant une fréquence cardiaque reçue depuis le moniteur de fréquence cardiaque (104) ;
sélectionner un premier et un deuxième signal audio ayant respectivement un tempo élevé et un tempo bas ;
le procédé étant **caractérisé en ce qu'**il comprend en outre :
le rendu en alternance des premier et deuxième signaux audio à un utilisateur, dans lequel un signal audio respectif est rendu à l'utilisateur correspondant à la première et à la deuxième valeur de paramètre cible, telles que déterminées par l'unité de traitement (102) utilisant le paramètre provenant de l'unité de détection (104) ; dans lequel le rendu comprend le rendu répété du premier signal audio à l'utilisateur jusqu'à ce que la fréquence cardiaque de l'utilisateur atteigne ou dépasse la première fréquence cardiaque cible puis le rendu du deuxième signal audio jusqu'à ce que la fréquence cardiaque de l'utilisateur atteigne ou tombe sous la deuxième fréquence cardiaque cible puis le rendu du premier signal audio.

9. Procédé selon la revendication 8, comprenant en outre l'étape consistant à, par un utilisateur, sélectionner les première et deuxième valeurs de paramètre cibles dans un groupe de valeurs de paramètre cibles prédéterminées ou une routine d'exercices programmés qui inclut les première et deuxième valeurs de paramètre cibles.

10. Procédé selon la revendication 8, dans lequel les signaux audio sont codés dans un format MP3, WAV, MPEG-4 ou WMA.

11. Procédé selon la revendication 8, comprenant en outre l'étape consistant à sélectionner un troisième et/ou un quatrième signal audio ayant des tempos respectifs similaires aux premier et deuxième signaux audio.

12. Procédé selon la revendication 9, comprenant en outre l'étape consistant à, à un moment prédéterminé, rendre les troisième et quatrième signaux audio à la place des premier et deuxième signaux audio respectivement.
